(19) Europäisches Patentamt
European Patent Office
Office européen des brevets

(11) EP 0 834 499 B1

(12) EUROPEAN PATENT SPECIFICATION

(45) Date of publication and mention of the grant of the patent:
**25.07.2001 Bulletin 2001/30**

(21) Application number: **96115814.4**

(22) Date of filing: **02.10.1996**

(51) Int Cl.[7]: **C07C 209/68**, C07C 231/12, C07C 63/64, C07C 43/215, C07C 43/285, C07C 5/09, C07B 35/02

(54) **Process for the preparation of aromatic vinyl compounds**

Verfahren zur Herstellung von aromatischen Vinylverbindungen

Procédé pour la préparation de composés vinyliques aromatiques

(84) Designated Contracting States:
**DE**

(43) Date of publication of application:
**08.04.1998 Bulletin 1998/15**

(73) Proprietor: **DAINIPPON INK AND CHEMICALS, INC.**
**Itabashi-ku Tokyo (JP)**

(72) Inventors:
- **Hartl,Helmut, Dr. Chem.**
  **D-13347 Berlin (DE)**
- **Frings,Rainer B.,Dr. Chem.**
  **D-12307 Berlin (DE)**
- **Pithart, Cornelia,Dr. Chem.**
  **D-14163 Berlin (DE)**

(74) Representative: **Kraus, Walter, Dr. et al**
**Patentanwälte Kraus, Weisert & Partner**
**Thomas-Wimmer-Ring 15**
**80539 München (DE)**

(56) References cited:

**EP-A- 0 091 101** **EP-A- 0 560 532**
**WO-A-93/07108** **DE-A- 4 211 126**

- **PATENT ABSTRACTS OF JAPAN vol. 009, no. 112 (C-281), 16 May 1985 & JP 60 004139 A (SHOWA DENKO KK), 10 January 1985,**
- **CHEMICAL ABSTRACTS, vol. 88, no. 21, 22 May 1978 Columbus, Ohio, US; abstract no. 151771v, DORFMAN, YA. A. ET AL.: "Selective hydrogenation of phenylacetylene ...." page 533; column 2; XP002025699 & TEZISY DOKL - VSES. KONF. KHIM. ATSETILENA, 5TH, 1975, page 141**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 834 499 B1

Printed by Jouve, 75001 PARIS (FR)

## Description

This invention relates to a process for preparing aromatic vinyl compounds. Aromatic vinyl compounds which contain additional functional groups, such as $-NH_2$, -COOH or OH for example, constitute valuable starting materials or intermediates for high-grade industrial materials. Thus polyimides which are resistant to high temperatures can be obtained from aminostyrene, for example, whilst hydroxystyrene and derivatives thereof result in polymers which, on account of their properties, can be used for electronic purposes and for the production of printing plates.

The production of these aromatic vinyl compounds, such as functional styrene derivatives for example, is still encumbered with various defects and disadvantages. Thus in many cases substances have to be used which are harmful ecologically and as regards industrial hygiene, and which necessitate a high expenditure on protective measures for industrial operations and a high expenditure on the disposal of problematical by-products and waste products.

Moreover, multi-step reactions are involved in many cases, the overall yield from which is not always within an order of magnitude which is economically desirable. In addition, the reaction conditions of many production processes cause problems time after time.

Prior art preparation processes essentially comprise four basic methods:

1. the Wittig reaction,

2. Heck olefination with ethylene,

3. dehydrogenation of alkyl phenyl derivatives, and

4. dehydration of 1-hydroxy-1-phenylethanes.

All these basic methods have considerable disadvantages. Thus, for example, in the synthesis of olefines by the Wittig reaction an alkyl bromide is first reacted with triphenyl phosphine to form a phosphorylide, which is subsequently treated with an aldehyde. Formaldehyde, which is harmful as regards industrial hygiene, is used for this purpose for example (see EP-OS 600 367). Apart from this, equimolar amounts of triphenylphosphine oxide are formed, which can give rise to problems in the course of the process.

Heck olefination with ethylene also has two crucial disadvantages. Firstly, the reaction has to be conducted under pressure, and secondly the yields are generally only high enough for industrial purposes when aromatic iodine compounds are used, and also under some circumstances when aromatic bromine compounds are used. Even a new variant of the Heck reaction which has been described by M. Beller et al. (see M. Beller, H. Fischer, K. Kühlein, Tetrahedron Lett. 1994, 35 (47), 8773-76) is not accompanied by any significant advantages, since organic nitrites, which are harmful as regards industrial hygiene, are used in order to obtain the requisite diazonium salt.

The dehydrogenation of alkylphenyl derivatives, as described in JP-OS 6363634 for example, can also cause problems in the progress of the process on account of the high temperatures of up to 550°C which are necessary for this purpose. This is also the situation for the dehydration of 1-hydroxy-1-phenylethanes, which is described in EP-OS 307 751 for example. Furthermore, phenylhydroxyethane first has to be prepared from acetophenones by suitable reactions.

A process for the selective hydrogenation of aromatic acetylene compounds present as impurities in vinyl-aromatic compounds without substantial loss of vinyl-aromatic compound comprising adding hydrogen and an inert gas to a liquid phase vinyl-aromatic compound and contacting the aromatic acetylene compound with hydrogen in the presence of a selective hydrogenation catalyst, wherein the partial hydrogen pressure is from about 0.001 to about 0.05 bar is described in WO93/07108. Said impurities are phenylacetylene and alkyl- or vinyl-substituted phenylacetylenes which are present in the vinyl-aromatic compounds in amounts of up to about 5000 ppm. The hydrogenation of aromatic acetylene compounds substituted with functional groups other than alkyl or vinyl is not described in WO 93/07108.

A method for preparing a cis-olefin of the formula $R_1$-CH=CH-$R_2$ by reducing an alkyne of the formula $R_1$-C≡C-$R_2$ with formic acid in the presence of a homogeneous palladium catalyst is described in EP 0 560 532. $R_1$ and $R_2$ are independently selected from the group consisting of a hydrogen atom, ester group, substituted silyl group, carboxyl group, cyano group, aliphatic C1-C20 hydrocarbon group, and phenyl group.

The hydrogenation of acetylenic compounds in the presence of a homogeneous palladium salt catalyst is described in DE-A-42 11 126. This document, however, does not relate to the preparation of aromatic vinyl compounds substituted by one or more functional groups.

The purification of styrene by selective hydrogenation of phenylacetylene present as an impurity is described in EP-A-0 091 101 and JP-A-60 004 139.

Finally, the hydrogenation of unsubstituted phenylacetylene to styrene using a homogeneous platinum-tin

chloride complex catalyst is referred to in Chem. Abs., Vol. 88, Abstract No. 151771.

For all these reasons, functional aromatic vinyl compounds can currently be produced only in relatively small amounts, if at all. Due to their high production cost, they are therefore too expensive for further processing, to produce products for air and space travel for example.

The object which underlies the present invention is therefore to provide a process which considerably simplifies access to functional aromatic vinyl compounds, which can be employed in a versatile manner for the preparation of different substituted styrene derivatives, and which results in technically pure products without costly process measures.

This object is achieved by the invention.

The present invention relates to a process for preparing aromatic vinyl compounds of general formula (I)

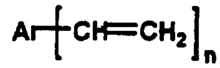

(I)

where n is an integer from 1 to 4 and Ar represents a group containing at least one aromatic ring and, apart from being directly attached to the vinyl group(s), is substituted by one or more functional groups, selected from the group consisting of halogen, -COOR, -OR, NRR', -NR-CO-OR', SR, $-SO_2R$, $-SO_3R$, -COR, -CONRR' or -NR-CO-R', where R and R', independently of each other, represent hydrogen, $C_1$-$C_{18}$ alkyl, $C_1$-$C_{18}$ alkyloxy, aryl or aralkyl,
which is characterised in that an aromatic acetylene compound of general formula (II)

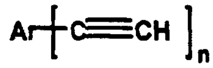

(II)

where n and Ar are as defined above, is selectively hydrogenated at the triple bond on a heterogeneous noble metal catalyst in a hydrogen atmosphere at a hydrogen pressure of 1 to 10 bar.

The process according to the invention is based on the selective, catalytic hydrogenation, which is effected at the triple bond, of functional aromatic ethynyl compounds, such as those which are readily accessible in high yields by the reaction of suitable starting compounds, such as substituted aromatic bromine compounds for example, with 2-hydroxy-2-methyl-1-butyltin. The synthesis of these ethynyl compounds can be effected analogously to the process described by A. P. Melissaris and M. H. Litt - J. Org. Chem. 1994, 59, 5818-21.

In general formula (I), Ar denotes a group containing at least one aromatic ring, for example a phenyl, naphthyl, anthracenyl, phenanthrenyl, fluorenyl, biphenyl, diphenylmethane, diphenylsulphone, oxadiphenyl or 2,2-diphenylpropane group. Ar preferably denotes a phenyl, naphthyl, anthracenyl, biphenyl or oxodiphenyl group. n is an integer within the range from 1 to 4, i.e. n may assume. the values 1, 2, 3 or 4. Compounds are preferred in which n is an integer from 1 to 3, and compounds in which n is the integer 1 or 2 are particularly preferred.

One preferred embodiment of the process according to the invention is characterised in that, for the preparation of functional styrene derivatives of general formula (III)

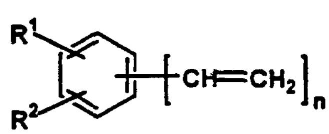

(III)

where n is an integer from 1 to 3 and $R^1$ and $R^2$, independently of each other, each represent hydrogen, halogen, or one of the following groups:
-COOR, -OR, NRR', -NR-CO-OR', SR, $-SO_2R$, $-SO_3R$, -COR, -CONRR' or -NR-CO-R', where R and R', independently of each other, represent hydrogen, $C_1$-$C_{18}$ alkyl, $C_1$-$C_{18}$ alkyloxy, aryl or aralkyl, a phenylacetylene derivative of general

formula (IV)

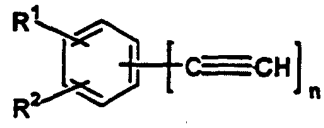

(IV)

where n, $R^1$ and $R^2$ are as defined above, is selectively hydrogenated at the triple bond on the noble metal catalyst with the proviso that not both $R^1$ and $R^2$ are hydrogen.

Examples of the aforementioned phenylacetylene derivative of general formula (IV) include: hydroxyphenylacetylene, ethynylbenzoic acid and alkali salts thereof, ethynylaniline, ethynylacetanilide, ethynylbenzoic acid methyl ester, ethynylanisole, 1-ethynyl(1-isobutoxy-ethoxy) benzene, ethynyl-chlorobenzene, benzyloxyphenylacetylene and N,N-dimethylaminophenylacetylene. Of these compounds, ethynylbenzoic acid and alkali salts thereof, ethynylaniline, 1-ethynyl(1-isobutoxy-ethoxy) benzene and hydroxyphenylacetylene are particularly preferred.

A further preferred embodiment of the process according to the invention is characterised in that, for the preparation of functional polynuclear aromatic vinyl compounds of general formula (V)

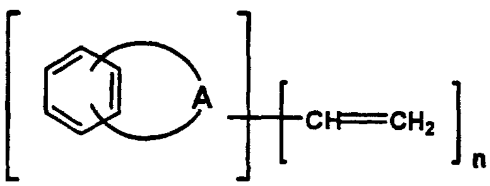

(V)

where A represents a benzene ring or a naphthalene, phenanthrene or anthracene system fused to the benzene ring, where the vinyl group(s) is (are) directly attached to any of the carbon atom(s) of the polynuclear aromatic moiety, where n is an integer from 1 to 4, and where the polynuclear aromatic ring system is substituted by one or more functional groups, selected from the group consisting of halogen, -COOR, -OR, NRR', -NR-CO-OR', SR, $-SO_2R$, $-SO_3R$, -COR, -CONRR' or -NR-CO-R', where R and R', independently of each other, represent hydrogen, $C_1$-$C_{18}$ alkyl, $C_1$-$C_{18}$ alkyloxy, aryl or aralkyl

an ethynyl derivative of general formula (VI)

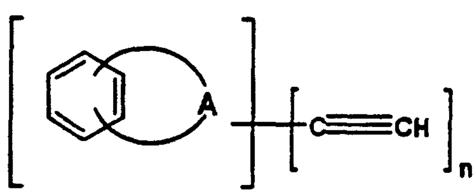

(VI)

where A and n are as defined above, is selectively hydrogenated at the triple bond on the noble metal catalyst

Examples of the aforementioned ethynyl derivative of general formula (VI) include: ethynylnaphthols, such as 6-ethynyl-2-naphthol for example, ethynylnaphthylamines such as 4-amino-1-ethynylnaphthalene for example, naphthalene carboxylic acids such as 4-ethynyl-1,8-naphthalenedicarboxylic acid for example, diethynylanthracenes such as 9,10-diethynylanthracene for example, and also 1-ethynylpyrene. Of these compounds, 9,10-diethynylanthracene, as well as ethynylnaphthols and naphthylamines, are particularly preferred.

Another preferred embodiment of the process according to the invention is characterised in that, for the preparation of functional polynuclear non-condensed aromatic vinyl compounds of general formula (VII)

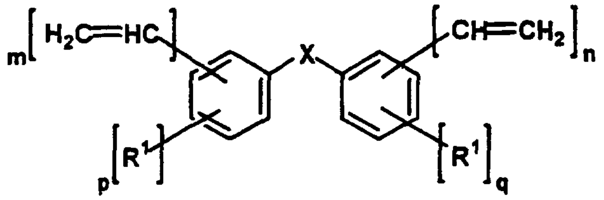

(VII)

where X represents either a single bond or one of the following groups:

-CR"R"'-, -CO-, $-SO_2-$, -SO-, $-C(CF_3)_2$, -S-, -O- or -NR"-, where R" and R"', independently of each other, represent hydrogen, $C_1$-$C_{18}$ alkyl, aryl or aralkyl, and where m and n are each an integer from 0 to 2, with the proviso that m + n at least = 1,

p and q each have a value of 0 to 2, with the proviso that p + q at least = 1,

and $R^1$ represents halogen or one of the following groups:

-COOR, -OR, -NRR', -NR-CO-OR', -SR, $-SO_2R$, $-SO_3R$, -CO-R, -CONRR' or -NK-CO-R', and where R and R', independently of each other, represent hydrogen, $C_1$-$C_{18}$ alkyl, $C_1$-$C_{18}$ alkyloxy, aryl or aralkyl,

an ethynyl derivative of general formula (VIII)

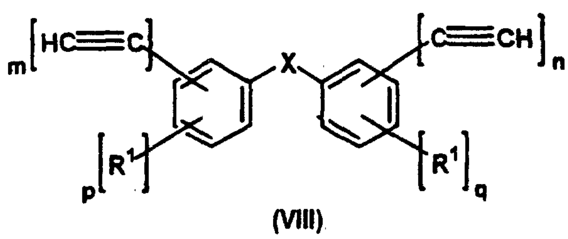

(VIII)

where X, m, n, p, q and $R^1$ are as defined above, is selectively hydrogenated at the triple bond on the noble metal catalyst.

Examples of the aforementioned ethynyl derivative of general formula (VIII) include: ethynyl- and diethynyl diphenyl ether, ethynyl- and diethynylbiphenyl, ethynyl- and diethynyldiphenyl sulphone, or ethynyl- and diethynylbenzophenone. Of these compounds, ethynyl- and diethynyldiphenyl ether and ethynyl- and diethynylbiphenyl are particularly preferred.

Hydrogenation of the triple bond is advantageously effected in a suitable solvent. Suitable solvents are organic and inorganic solvents and mixtures thereof in which the compounds to be hydrogenated dissolve.

Hydrogenation is most advantageously conducted in a solvent which is easily removed by simple operations, and which does not react irreversibly either with the functional group of the compound to be hydrogenated or with the support material of the catalyst. Suitable inorganic solvents in the sense of this invention are therefore water and aqueous solutions of alkalies, such as alkali and alkaline earth metal oxides and hydroxides for example, as well as aqueous solutions of mineral acids, such as hydrochloric acid for example.

Organic solvents which are particularly suitable include hydrocarbons such as hexane, petroleum ether and toluene for example, halogenated hydrocarbons such as chloroform and chlorobenzene for example, cyclic and alicyclic ethers such as tetrahydrofuran, dioxane and diethyl ether for example, as well as ketones such as acetone and methyl ethyl ketone for example. The following solvents may also be used: esters such as ethyl acetate for example, alcohols such as methanol, ethanol and isopropanol for example, ether alcohols such as diethylene glycol for example, amides such as N,N-dimethylformamide and N-methylpyrrolidone for example, and aliphatic and aromatic amines such as triethylamine and pyridine for example. Anhydrides, such as acetic anhydride for example, are also suitable. Moreover,

in principle any liquid compound or even any low melting compound which dissolves the acetylene compound to be hydrogenated to a sufficient extent can be used as a solvent. The preferred solvents are petroleum ether, n-hexane, toluene, xylene, diethyl ether, ethylene glycol dimethyl ether, methanol, ethanol, isopropanol, acetone, methyl ethyl ketone, acetic acid, ethyl acetate, water, triethylamine, pyridine, N,N-dimethylformamide and dimethyl sulphoxide, of which petroleum ether, toluene, methanol, acetone, pyridine, water and dimethyl sulphoxide are particularly preferred.

The selectivity of the hydrogenation reaction, i.e. the ratio of olefine to alkane, is generally already very high without auxiliary process materials, but can still be improved in some cases by the addition of such auxiliary process materials. Materials which are generally suitable for this purpose are the customary nitrogen heterocycles such as quinoline for example, as has also been described by T. Fukuda and T. Kusama (Bull. Chem. Soc. Jpn. 1958, 31, 339) or by H. J. Ringold and co-workers also (J. Am. Chem. Soc. 1959, 81, 427), as well as by D. J. Cram et al., (J. Am. Chem. Soc. 1956, 78, 2518) and H. H. Inhoffen et al. (Liebigs Ann. Chem. 1950, 570, 54-72). Sulphur-containing compounds, such as dimethyl sulphide (C. Kemball in "Catalysis - Progress in Research", edited by F. Basolo and R. L. Burwell, Plenum Press, London, New York, 1973, 85) for example, and substances which act analogously can also successfully be used. Provided that these auxiliary process materials are liquids or low melting compounds, they can also be used as solvents. The preferred auxiliary process materials are quinoline, isoquinoline and pyridine; pyridine and quinoline are particularly preferred.

According to the invention. the hydrogenation of the acetylene compounds is effected on noble metal hydrogenation catalysts known in the art, such as those which are available commercially from various manufacturers worldwide. The catalyst is preferably a catalyst from the eighth sub-group of the periodic table. Examples of suitable hydrogenation catalysts include those comprising the metals rhodium, ruthenium and platinum, of which platinum is particularly preferred according to the invention. The noble metal catalyst can either be employed finely divided in the reaction system, i.e. without a support, or may be employed in precipitated form on a support. Examples of suitable support materials include calcium carbonate, barium sulphate, alumina and activated carbon, preferably calcium carbonate and barium sulphate. Catalysts of the type described above are insoluble in the reaction mixture, which makes separation and reuse of the catalysts easier.

In general, the content of noble metal catalyst on the support is 1 to 10 % by weight, preferably 2 to 5 % by weight. Lower metal contents significantly reduce the rate of hydrogenation and make it necessary to employ larger amounts of catalyst. In contrast, higher contents of noble metal can perceptibly impair the yield of the desired vinyl compound. In some cases it may also be advantageous if the noble metal is combined with small proportions of less noble metals, e.g. lead or tin.

The hydrogenation according to the invention is effected in a hydrogen atmosphere at hydrogen pressures of 1 bar to 10 bar, preferably at a hydrogen pressure of 1 bar. In the latter case pressure apparatuses do not need to be used, which enhances the simplicity of the process according to the invention. The reaction temperatures are generally within the range between the freezing point and the boiling point of the solvent or solvent mixture used, and are within the :range of 0°C to 80°C, preferably 20°C to 60°C. In individual cases the upper limit of the temperature is also determined by the solvent used, of course.

In principle, hydrogenation in the process according to the invention is effected in the manner known in the art, as is known from relevant textbooks, such as "Organikum", Deutscher Verlag der Wissenschaften Berlin, 1986, 16th revised Edition. The reaction is terminated when the theoretical amount of hydrogen is consumed.

The invention is explained in more detail with reference to the following examples.

## Examples

### Example 1

1.1 g 3-ethynylaniline, 15 g THF and 50 mg of 5 % Pd-$CaCO_3$ (Nippon Engelhard, Lot 58) were introduced in succession into a three-necked flask fitted with a stirrer. The flask was connected to a gas burette and the reaction was initiated by bringing the catalyst dispersed in the mixture into contact with the hydrogen atmosphere by vigorous stirring. The reaction was started at room temperature, but the temperature increased by about 10°C during the hydrogenation. After 228 ml of hydrogen had been consumed, the reaction was terminated. Yield of 3-aminostyrene: 94 %.

### Example 2

Hydrogenation was effected as in Example 1, except that 1.1 g 3-ethynylaniline, 14 g THF, 1 g quinoline and 50 mg of 5 % Pd-$CaCO_3$ (Nippon Engelhard, Lot 58) were introduced into the three-necked flask. Yield of 3-aminostyrene: 97 %.

**Example 3**

Hydrogenation was effected as in Example 1, except that 0.8 g 3-ethynylacetanilide, 7.5 g DMSO and 50 mg of 5 % Pd-$CaCO_3$ (Nippon Engelhard, Lot 58) were introduced into the three-necked flask. Yield of 3-acetaminostyrene: 98.1 %.

**Example 4**

Hydrogenation was effected as in Example 1, except that 0.73 g 4-ethynylbenzoic acid, 7.5 g water/$EtN_3$ (1: 1) and 50 mg of 5 % Pd-$BaSO_4$ (Nippon Engelhard, Lot 31) were introduced into the three-necked flask. Yield of 4-vinylbenzoic acid: 98.5 %.

**Example 5**

The procedure was as in Example 1, except that 0.8 g 3-ethynylacetanilide, 7.5 g isopropanol and 50 mg 5 % of Pd-$CaCO_3$ (Nippon Engelhard, Lot 58) were introduced into the reaction flask and the reaction temperature was 60°C. Yield of 3-acetaminostyrene: 95.9 %.

**Example 6**

Hydrogenation was effected as in Example 1, except that 1.17 g 1-ethynyl-4-[1-(3-methylbutoxy)ethoxy]-benzene (= 4-ethynylphenol blocked with isobutyl vinyl ether), 7.5 g pyridine and 50 nig of 5 % Pd-$CaCO_3$ (Nippon Engelhard, Lot 58) were introduced into the three-necked flask. Yield of 1-ethenyl-4-[1-(3-methylbutoxy)ethoxy]-benzene: 96.1 %.

**Example 7**

Hydrogenation was effected as in Example 1, except that 0.74 g 4-ethynylanisole, 7.5 g acetone and 50 mg of 2 % Pd-Sn-$CaCO_3$ (Nippon Engelhard, Lot 95) were introduced into the three-necked flask. Yield of 4-methoxystyrene: 94.7 %.

**Example 8**

Hydrogenation was effected as in Example 1, except that 0.54 g 4,4'-diethynyl diphenyl ether, 7.5 g acetone and 50 mg of 5 % Pd-$CaCO_3$ (Acros Chemicals) were introduced into the three-necked flask. The amount of hydrogen required for semi-hydrogenation was 112 ml. Yield of 4,4'-diethenyl diphenyl ether; 98.5 % (MS: m/e = 222 ($M^+$), 193, 178, 165, 128, 120, 91, 77, 51, 39).

**Example 9**

Hydrogenation was effected as in Example 1, except that 0.50 g 4,4'-diethynylbiphenyl, 7.5 g acetone and 50 mg of 5 % Pd-$CaCO_3$ (Acros Chemicals) were introduced into the three-necked flask. The amount of hydrogen required for semi-hydrogenation was 112 ml. Yield of 4,4'-diethenylbiphenyl; 99 % (MS: m/e = 206 ($M^+$), 178, 165, 152, 128, 103, 89, 76, 51).

**Example 10**

Hydrogenation was effected as in Example 1, except that 0.53 g 2,7-diethynylfluorene, 7.0 g toluene, 0.5 g quinoline and 50 mg of 5 % Pd-$CaCO_3$ (Acros Chemicals) were introduced into the three-necked flask. The amount of hydrogen required for semi-hydrogenation was 112 ml. Yield of 2,7-diethenylfluorene; 98.3 % (MS: m/e = 218 ($M^+$), 202, 176, 165, 139, 109, 94, 81, 63).

**Example 11**

Hydrogenation was effected as in Example 1, except that 0.38 g 1,4-diethynyl-2,5-dimethylbenzene, 7.0 g toluene, 0.5 g quinoline and 50 mg of 5 % Pd-$CaCO_3$ (Acros Chemicals) were introduced into the three-necked flask. The amount of hydrogen required for semi-hydrogenation was 112 ml. Yield of 1,4-diethenyl-2,5-dimethylbenzene; 96.7 % (MS: m/e = 158 ($M^+$), 143, 128, 115, 102, 91, 77, 65, 51).

**Example 12**

Hydrogenation was effected as in Example 1, except that 0.60 g 4-ethynylfluorobenzene, 7.5 g toluene and 50 mg of 5 % Pd-$CaCO_3$ (Acros Chemicals) were introduced into the three-necked flask. The amount of hydrogen required for semi-hydrogenation was 112 ml. Yield of 4-fluorostyrene; 92.7 % (MS: m/e = 122 ($M^+$), 108, 101, 96, 75, 61, 51).

## Claims

1. A process for preparing aromatic vinyl compounds of general formula (I)

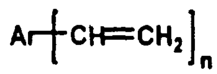

(I)

where n is an integer from 1 to 4 and Ar represents a group containing at least one aromatic ring and, apart from being directly attached to the vinyl group(s), is substituted by one or more functional groups, selected from the group consisting of halogen, -COOR, -OR, NRR', -NR-CO-OR', SR, -$SO_2$R, -$SO_3$R, -COR, -CONRR' or -NR-CO-R', where R and R', independently of each other, represent hydrogen, $C_1$-$C_{18}$ alkyl, $C_1$-$C_{18}$ alkyloxy, aryl or aralkyl, characterised in that an aromatic acetylene compound of general formula (II)

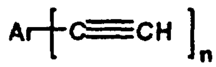

(II)

where n and Ar are as defined above, is selectively hydrogenated at the triple bond on a heterogeneous noble metal catalyst in a hydrogen atmosphere at a hydrogen pressure of 1 to 10 bar.

2. A process according to claim 1, characterised in that the Ar group in general formula (I) contains a phenyl, naphthyl, phenanthryl, anthracenyl, diphenyl or fluorenyl group.

3. A process according to claim 1, characterised in that, for the preparation of functional styrene derivatives of general formula (III)

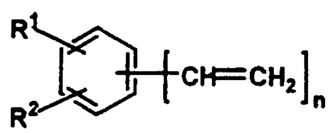

(III)

where n is an integer from 1 to 3 and $R^1$ and $R^2$, independently of each other, each represent hydrogen, halogen, or one of the following groups:
-COOR, -OR, NRR', -NR-CO-OR', SR, -$SO_2$R, -$SO_3$R, -COR, -CONRR' or -NR-CO-R', where R and R', independently of each other, represent hydrogen, $C_1$-$C_{18}$ alkyl, $C_1$-$C_{18}$ alkyloxy, aryl or aralkyl,
a phenylacetylene derivative of general formula (IV)

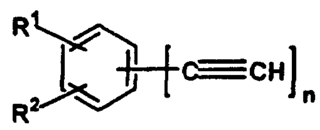

(IV)

where n, $R^1$ and $R^2$ are as defined above, is selectively hydrogenated at the triple bond on the noble metal catalyst with the proviso that not both $R^1$ and $R^2$ are hydrogen.

**4.** A process according to claim 1, characterised in that for the preparation of functional polynuclear aromatic vinyl compounds of general formula (V)

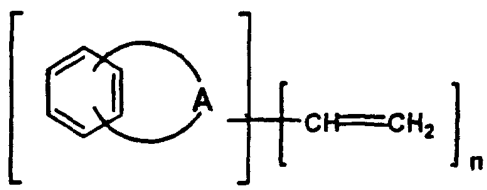

(V)

where A represents a benzene ring or a naphthalene, phenanthrene or anthracene system fused to the benzene ring, where the vinyl group(s) is (are) directly attached to any of the carbon atom(s) of the polynuclear aromatic moiety, where n is an integer from 1 to 4, and where the polynuclear aromatic ring system is substituted by one or more functional groups, selected from the group consisting of halogen, -COOR, -OR, NRR', -NR-CO-OR', SR, $-SO_2R$, $-SO_3R$, -COR, -CONRR' or -NR-CO-R', where R and R', independently of each other, represent hydrogen, $C_1$-$C_{18}$ alkyl, $C_1$-$C_{18}$ alkyloxy, aryl or aralkyl
an ethynyl derivative of general formula (VI)

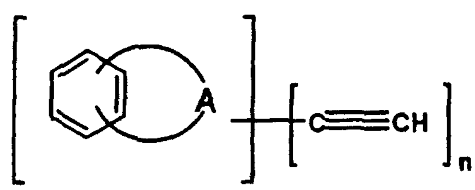

(VI)

where A and n are as defined above, is selectively hydrogenated at the triple bond on the noble metal catalyst.

**5.** A process according to claim 1, characterised in that, for the preparation of functional polynuclear non-condensed aromatic vinyl compounds of general formula (VII)

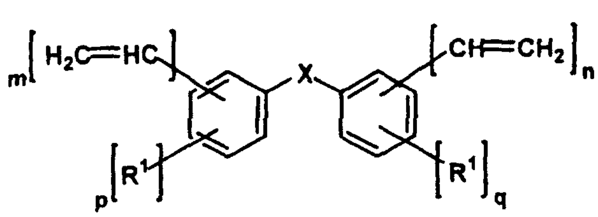

(VII)

where X represents either a single bond or one of the following groups:

-CR''R'''-, -CO-, $-SO_2-$, -SO-, $-C(CF_3)_2$, -S-, -O- or -NR''-, where R'' and R''', independently of each other, represent hydrogen, $C_1$-$C_{18}$ alkyl, aryl or aralkyl, and where m and n are each an integer from 0 to 2, with the proviso that m + n at least = 1,

p and q each have a value of 0 to 2, with the proviso that p + q at least = 1,

and $R^1$ represents halogen or one of the following groups:

-COOR, -OR, -NRR', -NR-CO-OR', -SR, $-SO_2R$, $-SO_3R$, -CO-R, -CONRR' or -NR-CO-R', and where R and R', independently of each other, represent hydrogen, $C_1$-$C_{18}$ alkyl, $C_1$-$C_{18}$ alkyloxy, aryl or aralkyl,

an ethynyl derivative of general formula (VIII)

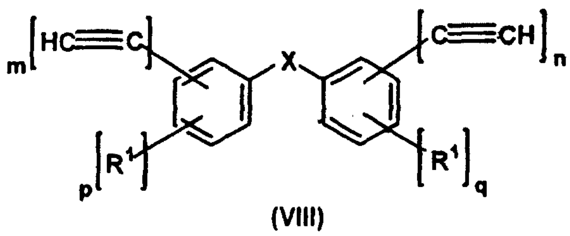

(VIII)

where X, m, n, p, q and $R^1$ are as defined above, is selectively hydrogenated at the triple bond on the noble metal catalyst.

## Patentansprüche

**1.** Verfahren zur Herstellung aromatischer Vinylverbindungen der allgemeinen Formel (I)

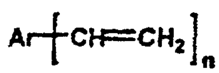

(I)

worin n eine ganze Zahl von 1 bis 4 bedeutet und Ar eine Gruppe, die mindestens einen aromatischen Ring enthält, und unabhängig davon, dass sie direkt an die Vinylgruppe(en) gebunden ist, durch eine oder mehrere funktionelle Gruppen, ausgewählt aus der Gruppe, bestehend aus Halogen, -COOR, -OR, NRR', -NR-CO-OR', SR, $-SO_2R$, $-SO_3R$, -COR, -CONRR' oder -NR-CO-R', substituiert ist, worin R und R' unabhängig voneinander Wasserstoff, $C_1$-$C_{18}$-Alkyl, $C_1$-$C_{18}$-Alkyloxy, Aryl oder Aralkyl bedeuten, dadurch **gekennzeichnet**, dass eine aromatische Acetylenverbindung der allgemeinen Formel (II)

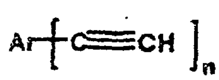

(II)

worin n und Ar die oben gegebenen Definitionen besitzen, selektiv an der Dreifachbindung an einem heterogenen Edelmetallkatalysator in Wasserstoffatmosphäre bei Wasserstoffdruck von 1 bis 10 bar hydriert wird.

**2.** Verfahren nach Anspruch 1, dadurch **gekennzeichnet**, dass die Ar-Gruppe in der allgemeinen Formel (I) eine Phenyl-, Naphthyl-, Phenanthryl-, Anthracenyl-, Diphenyl- oder Fluorenylgruppe enthält.

**3.** Verfahren nach Anspruch 1, dadurch **gekennzeichnet**, dass zur Herstellung eines funktionellen Styrolderivats der allgemeinen Formel (III)

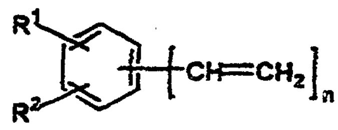

(III)

worin n eine ganze Zahl von 1 bis 3 bedeutet und $R^1$ und $R^2$ unabhängig voneinander je Wasserstoff, 1 Halogen oder eine der folgenden Gruppen bedeuten: -COOR, -OR, NRR', -NR-CO-OR', SR, $-SO_2R$, $-SO_3R$, -COR, -CONRR' oder -NR-CO-R' bedeuten, worin R und R' unabhängig voneinander Wasserstoff, $C_1$-$C_{18}$-Alkyl, $C_1$-$C_{18}$-Alkyloxy, Aryl oder Aralkyl bedeuten, ein Phenylacetylenderivat der allgemeinen Formel (IV)

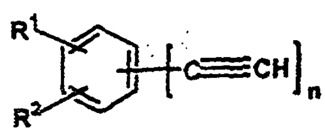

(IV)

worin n, $R^1$ und $R^2$ die oben gegebenen Definitionen besitzen, selektiv an der Dreifachbindung an dem Edelmetallkatalysator hydriert wird, mit der Maßgabe, dass $R^1$ und $R^2$ nicht beide Wasserstoff bedeuten.

**4.** Verfahren nach Anspruch 1, dadurch **gekennzeichnet**, dass zur Herstellung funktioneller polynuklearer aromatischer Vinylverbindungen der allgemeinen Formel (V)

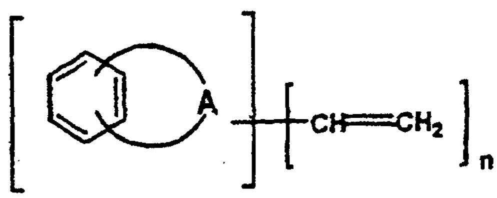

(V)

worin A ein Benzolring oder ein Naphthalin-, Phenanthren- oder Anthracensystem, kondensiert an den Benzolring, bedeutet, und wobei die Vinylgruppe(n) direkt an eines der Kohlenstoffatome der polynuklearen aromatischen Gruppierung gebunden ist (sind), wobei n eine ganze Zahl von 1 bis 4 bedeutet und wobei das polynukleare aromatische Ringsystem durch eine oder mehrere funktionelle Gruppen, ausgewählt aus der Gruppe, bestehend aus Halogen, -COOR, -OR, NRR', -NR-CO-OR', SR, $-SO_2R$, $-SO_3R$, -COR, -CONRR' oder -NR-CO-R', substituiert ist, wobei R und R' unabhängig voneinander Wasserstoff, $C_1$-$C_{18}$-Alkyl, $C_1$-$C_{18}$-Alkyloxy, Aryl oder Aralkyl bedeuten, ein Ethinylderivat der allgemeinen Formel (VI)

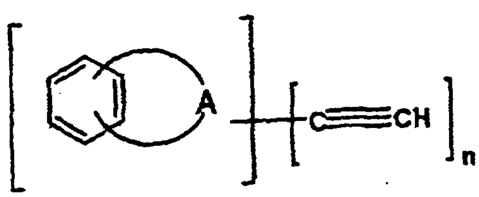

(VI)

worin A und n die oben gegebenen Definitionen besitzen, selektiv an der Dreifachbindung an dem Edelmetallkatalysator hydriert wird.

**5.** Verfahren nach Anspruch 1, dadurch **gekennzeichnet**, dass zur Herstellung von funktionellen polynuklearen nichtkondensierten aromatischen Vinylverbindungen der allgemeinen Formel (VII)

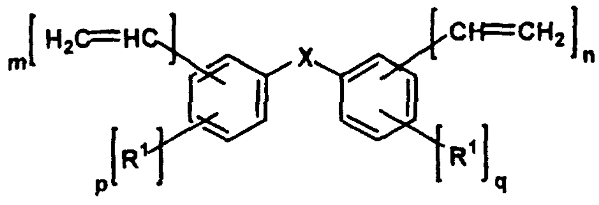

(VII)

worin X eine Einfachbindung oder eine der folgenden Gruppen bedeutet: -CR''R'' '-, -CO-, $-SO_2-$, -SO-, $-C(CF_3)_2$, -S-, -O- oder -NR''-, worin R'' und R''' unabhängig voneinander Wasserstoff, $C_1$-$C_{18}$-Alkyl, Aryl oder Aralkyl bedeuten und wobei m und n eine ganze Zahl von 0 bis 2 bedeuten, mit der Maßgabe, dass m + n mindestens 1 ist, p und q je einen Wert von 0 bis 2 besitzen, mit der Maßgabe, dass p + q mindestens 1 ist und $R^1$ Halogen oder eine der folgenden Gruppen bedeutet:-COOR, -OR, NRR', -NR-CO-OR', -SR, $-SO_2R$, $-SO_3R$, -CO-R, -CONRR' oder -NR-CO-R', und wobei R und R' unabhängig voneinander Wasserstoff, $C_1$-$C_{18}$-Alkyl, $C_1$-$C_{18}$-Alkyloxy, Aryl oder Aralkyl bedeuten, ein Ethinylderivat der allgemeinen Formel (VIII)

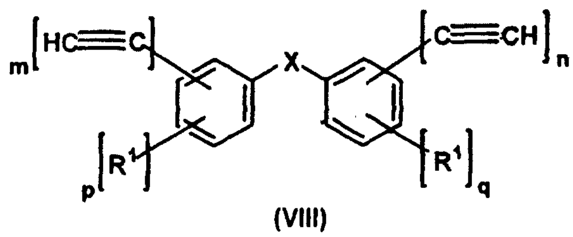

(VIII)

worin X, m, n, p, q und $R^1$ die oben gegebenen Definitionen besitzen, selektiv an der Dreifachbindung an einem Edelmetallkatalysator hydriert wird.

## Revendications

**1.** Procédé pour la préparation des composés vinyliques aromatiques de formule générale (I):

$$Ar\text{-}[\text{-}CH{=}CH_2]_n$$

(I)

dans laquelle n est un entier de 1 à 4 et Ar représente un groupe contenant au moins un cycle aromatique et, outre qu'il est directement attaché au ou aux groupes vinyliques, est substitué par un ou plusieurs groupes fonctionnels, choisis dans le groupe consistant en atome d'halogène, groupes -COOR, -OR, NRR', -NR-CO-OR', SR, $-SO_2R$, $-SO_3R$, -COR, -CONRR' ou -NR-CO-R', où R et R' représentent indépendamment l'un de l'autre un atome d'hydrogène, un groupe alkyle en $C_{1-18}$, alkyloxy en $C_{1-18}$, aryle ou aralkyle,
caractérisé en ce qu'un composé acétylène aromatique de formule générale (II) :

$$Ar\text{-}[\text{-}C{\cong}CH]_n$$

(II)

dans laquelle n et Ar sont tels que définis plus haut, est hydrogéné de façon sélective au niveau de la triple liaison sur un catalyseur hétérogène au métal noble dans une atmosphère d'hydrogène sous une pression d'hydrogène de 1 à 10 bar.

**2.** Procédé suivant la revendication 1, caractérisé en ce que le groupe Ar dans la formule générale (I) contient un groupe phényle, naphtyle, phénanthryle, anthracényle, diphényle ou fluorényle.

**3.** Procédé suivant la revendication 1, caractérisé en ce que, pour la préparation des dérivés fonctionnels du styrène de formule générale (III) :

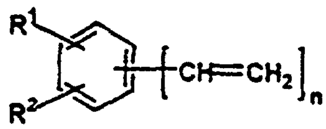

(III)

dans laquelle n est un entier de 1 à 3 et $R^1$ et $R^2$ représentent chacun indépendamment l'un de l'autre, un atome d'hydrogène, d'halogène ou l'un des groupes suivants : -COOR, -OR, NRR', -NR-CO-OR', SR, $-SO_2R$, $-SO_3R$, -COR, -CONRR' ou-NR-CO-R', où R et R' représentent indépendamment l'un de l'autre un atome d'hydrogène, un groupe alkyle en $C_{1-18}$, alkyloxy en $C_{1-18}$, aryle ou aralkyle,
un dérivé de phénylacétylène de formule générale (IV):

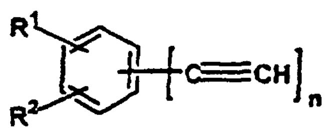

(IV)

dans laquelle n, $R^1$ et $R^2$ sont tels que définis plus haut, est hydrogéné de façon sélective au niveau de la triple liaison sur le catalyseur au métal noble à condition que $R^1$ et $R^2$ ne soient pas tous les deux un atome d'hydrogène.

**4.** Procédé suivant la revendication 1, caractérisé en ce que, pour la préparation des dérivés vinyliques aromatiques polynucléaires fonctionnels de formule générale (V):

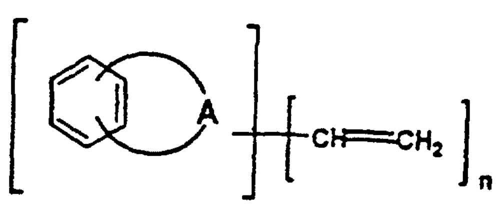

(V)

dans laquelle A représente un cycle benzène ou un système naphtalène, phénanthrène ou anthracène condensé

avec le cycle benzène, où le ou les groupes vinyliques sont directement attachés à l'un quelconque du ou des atomes de carbone de la partie aromatique polynucléaire, où n est un entier de 1 à 4, et où le système cyclique aromatique polynucléaire est substitué par un ou plusieurs groupes fonctionnels, choisis dans le groupe consistant en atome d'halogène, groupes -COOR, -OR, NRR', -NR-CO-OR', SR, $-SO_2R$, $-SO_3R$, -COR, -CONRR' ou -NR-CO-R', où R et R' représentent indépendamment l'un de l'autre, un atome d'hydrogène, un groupe alkyle en $C_{1-18}$, alkyloxy en $C_{1-18,}$ aryle ou aralkyle,
un dérivé éthynyle de formule générale (VI) :

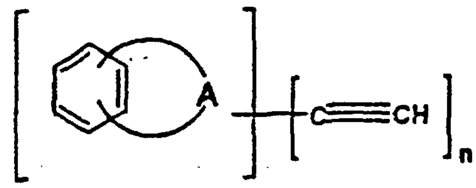

(VI)

dans laquelle A et n sont tels que définis plus haut, est hydrogéné de façon sélective au niveau de la triple liaison sur le catalyseur au métal noble.

**5.** Procédé suivant la revendication 1, caractérisé en ce que, pour la préparation des composés vinyliques aromatiques non condensés polynucléaires fonctionnels de formule générale (VII) :

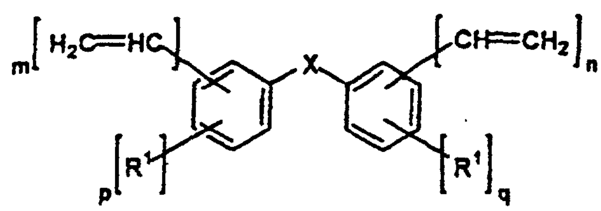

(VII)

dans laquelle X représente soit une simple liaison, soit l'un des groupes suivants : -CR''R'''-, -CO-, $-SO_2-$, -SO-, $-C(CF_3)_2$, -S-, -O- ou -NR''-, où R'' et R''' représentent indépendamment l'un de l'autre, un atome d'hydrogène, un groupe alkyle en $C_{1-18}$, aryle ou aralkyle, et où m et n sont chacun un entier de 0 à 2, à condition que m+n soit au moins égal à 1, p et q ont chacun une valeur de 0 à 2 à condition que p+q soit au moins égal à 1, et $R^1$ représente un atome d'halogène ou l'un des groupes suivants : -COOR, -OR, NRR', -NR-CO-OR', SR, $-SO_2R$, $-SO_3R$, -COR, -CONRR' ou -NR-CO-R', et où R et R' représentent indépendamment l'un de l'autre un atome d'hydrogène, un groupe alkyle en $C_{1-18,}$ alkyloxy en $C_{1-18,}$ aryle ou aralkyle,
un dérivé éthynyle de formule générale (VIII) :

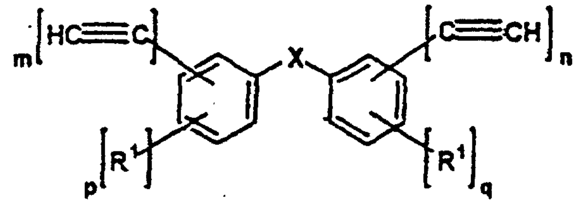

(VIII)

dans laquelle X, m, n, p, q et $R^1$ sont tels que définis plus haut, est hydrogéné de façon sélective au niveau de la triple liaison sur le catalyseur au métal noble.